# EUROPÄISCHE PATENTSCHRIFT

(11) **EP 1 509 597 B1**
(45) Veröffentlichungstag und Bekanntmachung des Hinweises auf die Patenterteilung: **30.12.2009**
(21) Anmeldenummer: 03756966.2
(22) Anmeldetag: 05.06.2003
(51) Int. Cl.: C12N 5/00

(54) **Verfahren zur Isolierung von mesenchymalen Stammzellen des Haarfollikels**
Method for isolating hair follicle mesenchymal stem cells
Procédé d'isolation de cellules souches mesenchymateuses du follicule pileux

(30) Priorität: 05.06.2002 DE 10224982
(43) Veröffentlichungstag der Anmeldung: 02.03.2005
(73) Patentinhaber: Hoffmann, Rolf, 79117 Freiburg (DE)
(72) Erfinder: HOFFMANN, Rolf, 79117 Freiburg (DE); MCELWEE, Kevin J.,, Wetherby ,West Yorkshire LS22 7TG (GB)
(74) Vertreter: Dehmel, Albrecht
(86) Internationale Anmeldenummer: PCT/DE2003/001863
(87) Internationale Veröffentlichungsnummer: WO 2003/104443

(56) Entgegenhaltungen:
- WO-A-02/40645
- WO-A-95/01423
- WO-A1-99/03505
- WO-A2-01/32840
- DATABASE BIOSIS [Online] BIOSCIENCES INFORMATION SERVICE, PHILADELPHIA, PA, US; Dezember 1997 (1997-12) WU JINJIN ET AL.,: "An efficient method for isolation and cultivation of human scalp dermal papilla cells" Database accession no. PREV199800181268 XP002265629
- WU JINJIN ET AL: 'An efficient method for isolation and cultivation of human scalp dermal papilla cells' ZHONGHUA PIFUKE ZAZHI Bd. 30, Nr. 6, Seiten 383 - 385
- REYNOLDS AMANDA J. ET AL: 'Hair matrix germinative epidermal cells confer follicle-inducing capabilities on dermal sheath and high passage papilla cells' DEVELOPMENT Bd. 122, Nr. 10, Seiten 3085 - 3094

## Beschreibung

Die vorliegende Erfindung betrifft ein Verfahren zur Isolierung von mesenchymalen Stammzellen des Haarfollikels.

Unter Aussparung von Schleimhaut, Handtellern und Fußsohlen finden sich am gesamten Integument des Menschen Haarfollikel, die eine in sich geschlossene komplexe Funktionseinheit, ein Miniaturorgan, darstellen. Topographisch-anatomisch werden vier Anteile unterschieden a) *Infundibulum:* Abschnitt zwischen Haarfollikelostium und Einmündung der Talgdrüse in den Haarkanal; b) *Isthmus:* Abschnitt zwischen Talgdrüseneinmündung und der Ansatzstelle des M. arector pili; c) *Infrainfundibulum (supi-abulbärer Anteil):* Abschnitt zwischen der Ansatzstelle des M. arrector pili bis zum Bulbus; und d) *Haarbulbus* einschließlich der Papille. Man schätzt, dass der behaarte Kopf etwa 100.000 Haarfollikel beheimatet, welche in Gruppen von 3-5 Haarfollikeln, sog. follikulären Einheiten, über die Kopfhaut verteilt sind. Diese follikulären Einheiten sind von einem feinen kollagenen Fasergeflecht umgeben und werden durch breitere Kollagenfasern voneinander getrennt.

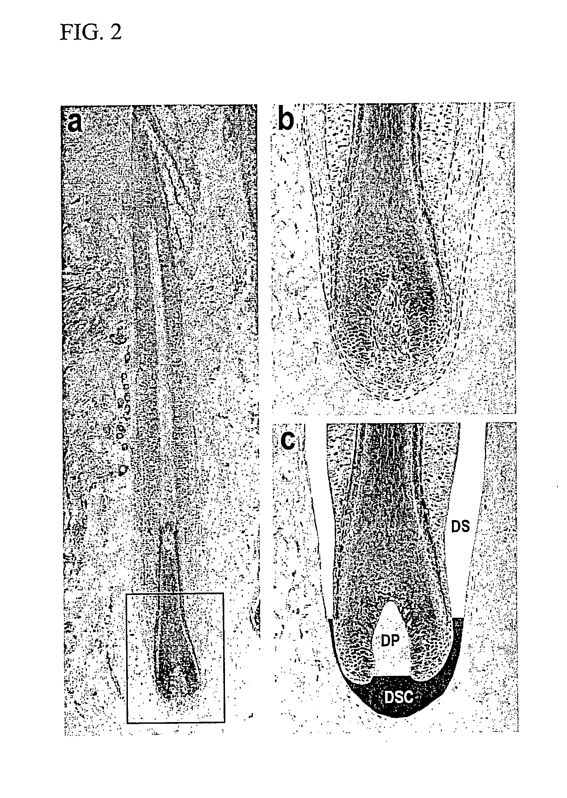

Der zwiebelförmige Haarbulbus bildet das proximale Ende des wachsenden Haarfollikels und reicht bei Terminalhaaren bis in das subkutane Fettgewebe (Fig. 2a, Kasten). Im Haarbulbus gelegene Haarmatrixzellen differenzieren aus und bilden dadurch den Schaft. Die Zellen der Matrix sind sog. "transit amplifying cells", d.h. eine Population von Zellen, welche nach einer Phase hochproliferativen Wachstums absterben. In den proximalen Haarbulbus hinein wölbt sich wie eine Zwiebel die dermale Haarpapille (Fig 2b und c, die durch ein feines Nerven- und Gefäßgeflecht versorgt wird. Die dermale Haarpapille unterscheidet sich von der Dermis dahingehend, dass sie in eine extrazelluläre Matrix eingebettet ist, die in ihrer Zusammensetzung einer Basalmembran gleicht. Während der Wachstumsphase des Haarfollikels (sog. Anagen) können somit einzelne Fibroblasten der dermalen Haarpapille über Zellfortsätze in direkten Kontakt zu den Matrixkeratinozyten treten. Über der Kuppe der dermalen Papille sind Melanozyten angesiedelt, die in Abhängigkeit vom Haarzyklus ab dem Anagenstadium IV bis zu Beginn des Katagens (Regressionsphase), Melanogeneseaktivität aufweisen. Durch die spezialisierten Haarpapillenzellen wird die Aktivität der Matrixkeratinozyten durch morphogene und mitogene Signale reguliert. Kommt es in diesem Haarsegment zu Störungen, so wird die Wachstumsphase (Anagen) beendet und der Follikel tritt in die Regressionsphase, das Katagen, ein. Daraus wird deutlich, dass Prozesse, welche die Matrixkeratinozyten und die darüberliegende Wulstregion (bulge area) zerstören, zu irreversiblem Haarverlust führen, während Noxen, die lediglich die Funktion der Papillenzellen beeinflussen, Die Größe der Papille die Dicke der zu bildenden Haarschäfte determiniert. Daher werden die größten Haarpapillen in Barthaaren und zunehmend kleinere bei einer androgenetischen Alopezie gefunden.

Die bindegewebige Haarwurzelscheide (DS = dermal sheath), der sogenannte Haarbalg, besteht aus zwei Schichten kollagener Fasern, wobei die innere zirkulär um den Haarschaft angeordnet ist. Der dickere äußere Teil der mesenchymalen Wurzelscheide enthält parallel zum Haarschaft verlaufende kollagene und elastische Fasern. Zusätzlich finden sich als Zeichen der taktilen Funktion des Haares Nervenfaserringgeflechte, die bis auf die Glasmembran vordringen. Die mesenchymale Haarwurzelscheide geht fließend am proximalen Ende in das sog. Papillenpolster über.

Der Haarschaft wird hülsenförmig von teleskopartig ineinandergeschobenen epithelialen Wurzelscheiden umgeben. In der Höhe der intrafollikulären Haarschaftbildung und - pigmentierung lassen sich im Querschnitt leicht eine innere Wurzelscheide (IWS) und eine äußere Wurzelscheide (ÄWS) abgrenzen. Die IWS wird aus der äußeren, meist zweilagigen Henle-Schicht, der mittleren mehrlagigen Huxley-Schicht, sowie der Kutikula gebildet. Alle drei Schichten entstehen aus den am äußeren Bulbusrand gelegenen Matrixzellen. Während die äußere Wurzelscheide kontinuierlich in die Basalzellschicht der Epidermis übergeht, bricht die IWS etwa auf Höhe des Infundibulum ab. Daher zeigt der distale Übergang zur epidermalen Auskleidung des Haarfollikelostiums eine epidermale Verhornung. Direkt unterhalb der Talgdrüsenmündung grenzt die ÄWS an die IWS. Ein wichtiger Ort der ÄWS ist die Ansatzstelle des M. arrector pili, der sogenannte Wulst. In dieser Region sowie proximal davon vermutet man den Sitz der epithelialen Stammzellen des Haarfollikels. Mittels eines Horizontalschnitts auf Höhe des Isthmus erkennt man Terminalhaare anhand des im Vergleich zur IWS dickeren Haarschaftes und Vellushaare an feinen Haarschäften, die dünner als die IWS sind.

Der Haarfollikel wird aus zwei primären Zellarten zusammengesetzt. Die eine Zellart wird zu Beginn der Haarfollikelmorphogenese aus dem embryonalen Ektoderm / der Epidermis, und der andere wird aus mesodermalen Anteilen rekrutiert. Während die epithelialen Stammzellen des Haarfollikels sich maßgeblich innerhalb der sogenannten "Wulst"-Region (Ansatz des Haarbalgmuskels) des Haarfollikels befinden, war die gültige Lehrmeinung, dass die mesenchymalen Stammzellen in der dermalen Papille residieren. Diesbezüglich haben vorangehende Untersuchungen gezeigt, dass präparierte dermale Papillen in haarlose Hautsegmente implantiert werden können, und dass dies die Bildung von neuen Haarfollikeln induziert (Oliver 1970, Jahoda et al 1984, Reynolds et al 1999). Dabei bestimmt der Entnahmeort der Papillenzellen die Art der gebildeten Haare (z.B. aus Schnurrhaarpapillen werden in einem Mäuseohr wieder Schnurrhaare). Es können auch dermale Papillen (DP) in Nährmedien gesetzt werden, um die Zellzahl zu erhöhen. Diese kultivierten DP-Zellen können in haarlose Hautareale (z.B. Handteller) implantiert werden, um sind sogar hier in der Lage, die Bildung von neuen Haarfollikeln einzuleiten (Messenger 1984). Die DP-Zellen sind zwar in der Lage, Haare zu induzieren, repopulieren aber nicht die DSC- oder DS-Region. DSC bedeutet "dermal sheath cup" und gibt die Lage der Zellen wieder. Ferner haben die durch die DP-Zellen gebildeten Haare nur eine kurze Lebensdauer.

In WO99/03505 wird gezeigt, dass mesenchymalen Stammzellen in der Haarschale anwesend sind. WO99/03505 lehrt aber nicht, dass die mesenchymalen Stammzellen der Haarschale eine alkalische Phosphatase-Aktivität aufweisen.

Haarverlust ist zum einen ein Teil des Alterungsprozesses (senile Alopezie), Resultat aktiver Krankheitsmechanismen wie bei der androgenetischen Alopezie, Alopecia areata und vernarbenden/traumatischen Alopezien, oder die Folge einer Chemotherapie. Haarverlust wird von der Gesellschaft im Allgemeinen in einem negativen Licht betrachtet. Das starke Verlangen nach Therapien, um Haarverlust vorzubeugen oder um Haare zu ersetzen, hat zu der Entwicklung von einer Vielzahl von verschiedenen Medikamenten, Produkten und Techniken geführt. In der haarbiologischen Forschung ist die dermale Papille innerhalb der Haarfollikeleinheit als eine Schlüsselstruktur identifiziert worden, die die Entwicklung und Differenzierung des Haarfollikels in der Embryogenese bestimmt, und die sowohl das Wachsen der Haarfaser als auch den Haarfollikelzyklus kontrolliert. Bei vielen Haarverlustkrankheiten, einschließlich der bekanntesten, der androgenetischen Alopezie, wird die dermale Papille von äußeren Faktoren beeinflusst, die dazu führen, dass die dermale Papille die Einheit des Haarfollikels nicht aufrecht erhalten kann. Teilweise scheint das auf eine Reduzierung der Größe und des Verlustes von Zellen aus der dermalen Papille zurückzuführen zu sein. Die reduzierte Größe der dermalen Papille wird direkt mit der reduzierten Größe von Haarfollikeln in Verbindung gebracht.

Vereinfacht lassen sich Haarerkrankungen als "zuviel" (Hypertrichose/Hirsutismus) oder als "zu wenig" (alle Formen der Alopezie wie Alopecia areata, androgenetische Alopezie, Pseudopelade Brocq, Alopezien bei Lichen planopilaris, Lupus erythematodes, angeborene Hypotrichosen und Atrichien (papulöse Atrichie u.a.), diffuser Haarverlust im Rahmen einer Stoffwechselerkrankung wie z.B. eine Schilddrüsenfunktionsstörung, Alopezien nach Verbrennung oder Traumen, Alopezien nach einer Chemotherapie uam.) definieren. Für diese unterschiedlichen Alopezien stehen lediglich für die androgenetische Alopezie zwei zugelassene Wirkstoffe (Finasterid, Minoxidil) zur Verfügung. Kein Wirkstoff beeinflusst die Stammzellen, und kein Wirkstoff kann in allen Fällen kosmetisch akzeptablen Haarwuchs garantieren. Die Behandlung der Hypertrichosen erfolgt im wesentlichen physikalisch, d.h. Zerstörung des Haarfollikels durch Lasertherapie. Auch hier wäre die Hemmung der Stammzellfunktion wirkungsvoller.

Es besteht daher ein begründeter Bedarf an Mitteln, um zu geringes Haarwachstum zu behandeln.

Die Aufgabe der vorliegenden Erfindung wird durch den in den Patentansprüchen beschriebenen Gegenstand gelöst.

Die nachfolgenden Figuren erläutern die Erfindung.

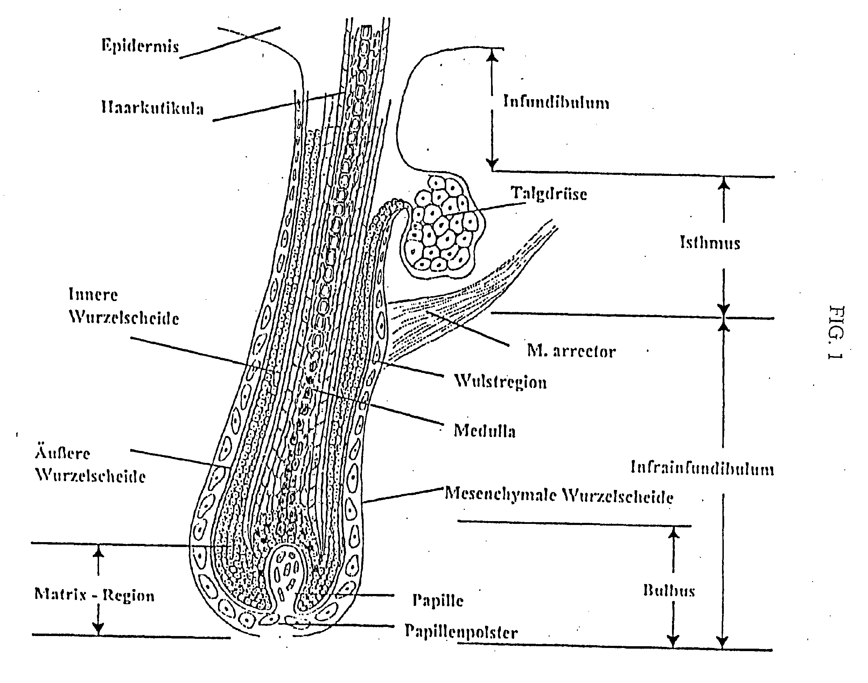

Figur 1 ist eine schematische Darstellung eines Terminalhaarfollikels.
Figur 2A zeigt im histologischen Schnitt ein Anagenhaar. Der Rahmen gibt den in Figur 2B und C dargestellten Ausschnitt wieder. DSC bedeutet "dermal sheath cup" und gibt die Lage der Zellen wieder. Die DSC-Zellen sind eindeutig anatomisch-topographisch anhand ihrer Lage im Haarfollikel definiert und befinden sich am unter Pol des Haarbulbus in einer tassenartigen den Haarbulbus umhüllenden Lage. DP bedeutet "dermale Papille". DS bedeutet "dermal sheath".

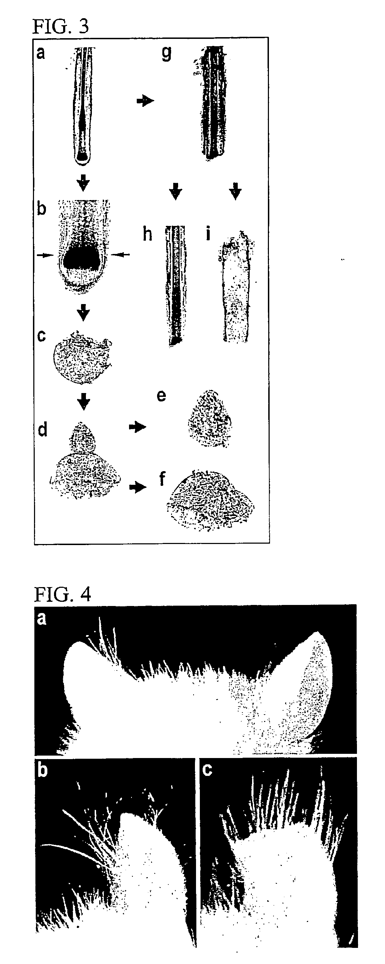

Figur 3 (a) bis (i) illustriert die einzelnen Stadien eines Dissektionsschemas zur Gewinnung von Haartassenzellen (DSC). Intakte Anagenhaare (a) werden unter dem Stereomikroskop präpariert. Die vergrößerte Ansicht in (b) zeigt die Dissektionsebene: am oberen Pol der pigmentierten Zone erfolgt ein Querschnitt durch das Haar; die tassenförmig angelagerte Haarschale (DSC) lässt sich mit der dermalen Haarpapille abstreifen (c). Dieses Gewebsstück wird umgestülpt (d) und die dermale Papille (e) von der Haarschale (f) getrennt; es verbleiben die epithelialen Anteile (h) des Haarfollikels und die Bindegewebshülle (i) = dermal sheath = DS.
Figur 4 (a) bis (c) zeigt in einer Darstellung das Ergebnis einer Implantation von DSC-Zellen in ein Mäusohr. Die isolierten DSC-Gewebe wurden in der Zellkultur vermehrt und Zellen wurden in ein zunächst haarloses Mäuseohr implantiert. Nach Implantation von Schnurrhaar-DSC-Zellen in das rechte Ohr wachsen Schnurrhaare aus diesem Mäuseohr. Das linke, unbehandelte Ohr zeigt kein Haarwachtum von Schnurrhaaren. Die Fig. 4b und 4c zeigen jeweilige Vergrösserungen.

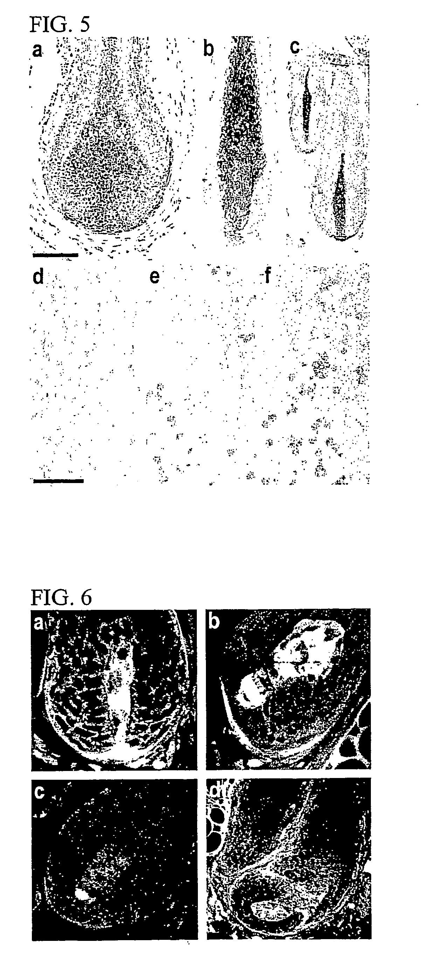

Figur 5 (a) bis (f) zeigt in Histogrammen die alkalischen Phosphatase Aktivität. Diese Figur zeigt die starke Expression der alkalischen Phosphatase in der dermalen Haarpapille, während die DSC-Zellen nur eine schwache Expression aufweisen. Die Expression endet abrupt im Übergang von der DSC zur DS (Fig. 3b und c, Fig. 5 a-c). In vitro zeigen kultivierte Zellen der DP (Fig. 5f) und DSC (Fig. 5e) ein identisches Wachstumsmuster, welches zur Bildung von sog. Pseudopapillen neigt. Die Zellen der DS zu einem Wachstumsmuster mit stark elongierten Zellen, in einer fischfleischartigen (Fig. 5d) Anordnung neigen. Die Zellen der DP (Fig. 5f) zeigen eine starke, die der DSC (Fig. 5e) eine schwache und die der DS (Fig. 5d) keine Aktivität der alkalischen Phosphatase.
Figur 6 zeigt induzierte und repopulierte dermale Haarpapillen nach Implantation von DSC-Zellen. Fluoreszierende DSC-Zellen aus TgN-GFPX-Mäusen wurden wie beschrieben kultiviert und in SCID-Mäuseohren implantiert. Nach 6 Monaten zeigte sich ein deutliches, neues Haarwachstum (Fig. 4). Im konfokalen Lasermikroskop fanden sich nach Implantation fluoreszierende Zellen sowohl in der dermalen Haarpapille als auch in der DSC-Region und zum Teil im dermal sheath (a, b). während zum Teil alle Zellen der neu gebildeten Papille ein Fluoreszenz aufwiesen, zeigen andere vereinzelt fluoreszierende Zellen (c, d), als Indiz dafür, dass die DSC-Zellen eine vorbestehende Papille repopulieren können, um damit ein dickeres Haar zu bilden.

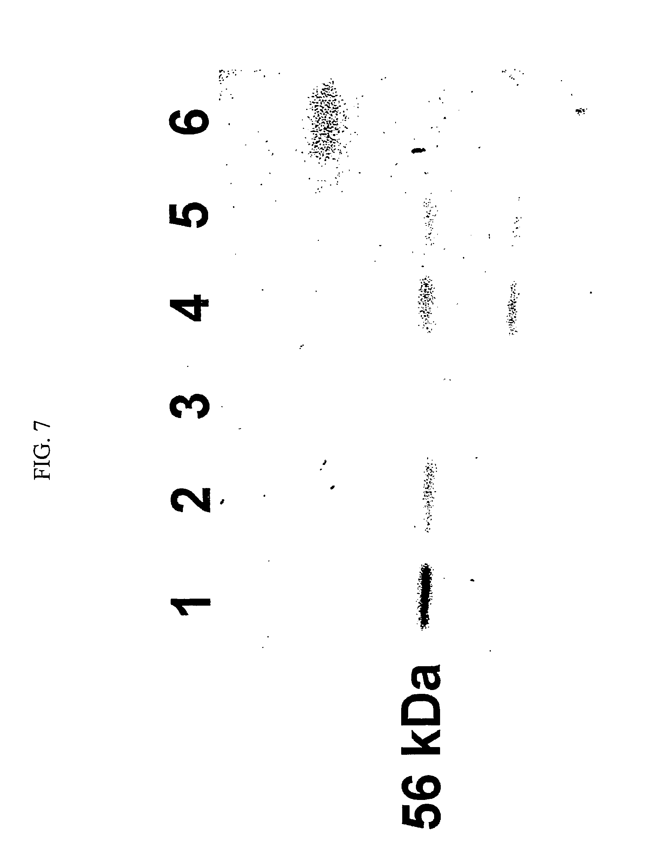

Figur 7 zeigt das Ergebnis eines Western blot für MSP-Protein: MSP. Proteinextrakte aus kultivierten Zellen der dermalen Haarpapille (Zytosol = Bahn 1, membrangebunden = Bahn 4); dermale Tassenzellen (Zytosol = Bahn 2, membrangebunden = Bahn 5) und follikuläre Fibroblasetn (Zytosol = Bahn 3, membrangebunden = Bahn 6) wurden chromatographisch aufgetrennt (SDS-PAGE: 12% Polyacrylamid) und auf eine Nylonmembran (Hybond ECL, Amersham Biosciences GmbH, Freiburg, Germany) geblottet. Die Membranen wurden blockiert mit 5% fettfreien Milchpulver und mit 0,5% Tween 20 (Sigma-Aldrich GmbH, Munich, Germany) in PBS gewachen. Ein polyklonaler Ziege anti-human MSP Antikörper gegen MSP (HGFL (N-19), sc-6088, Santa Cruz) wurde in einem ECL-Detektionssystem (Amersham) nach Angaben des Herstellers benutzt. Deutlich erkennt man die starke Bande bei 56kD insbesondere in den Zellen der dermalen Papille.

Die adulten mesenchymalen Stammzellen des Haarfollikels (DSC) haben die Eigenschaft ein Haarfollikel komplett neu zu bilden, in eine bestehende Haarpapille einzuwandern, einen Teil der dermalen Bindegewebshülle zu bilden und einer geringer alkalischen Phosphatase-Aktivität als Zellen der dermalen Papille. Vorzugsweise stammen die DSC-Zellen aus einem Säugetier, insbesondere aus einer Maus, Ratte, Kaninchen, Meerschwein, Ziege, Schwein, Rind oder Mensch. Die DSC-Zellen sind in der Lage bzw. besitzen die Eigenschaft, im Unterschied zu Zellen der follikulären Bindegewebshülle (DS) und der dermalen Papille (DP) ein Haarfollikel komplett neu zu bilden oder aber in eine bestehende Haarpapille einzuwandern, um damit ein größeres, bzw. dickeres Haar zu produzieren. Des weiteren sind diese Zellen in der Lage bzw. besitzen die Eigenschaft, einen Teil der dermalen Bindegewebshülle zu bilden. Diese zwei Charakteristika sind weder für DS-Zellen noch für die DP-Zellen gegeben. Die adulten mesenchymalen Stammzellen des Haarfollikels, im folgenden auch DSC-Stammzellen oder DSC-Zellen genannt, sind in einer tassenförmigen Anordnung um den unteren Pol des Haarbulbus (im folgenden auch Haarschale oder Haartasse genannt) zu finden sind, und sind daher Haartassenzellen genannt (dermal sheath cup = DSC) genannt worden. Der hier verwendete Begriff "adult" in Verbindung mit mesenchymalen Stammzellen bedeutet, dass es sich bei den mesenchymalen Stammzellen nicht um embryonale Stammzellen handelt sondern um aus adulten Organismen isolierte mesenchymale Stammzellen.

Die DSC-Zellen können biochemisch charakterisiert werden. Hierfür wurde deren Expression der alkalischen Phosphatase genutzt. Im Gegensatz zu den DP-Zellen zeigen die DSC-Zellen nur eine geringe alkalische Phosphatase-Aktivität. Die DP-Zellen sind dadurch gekennzeichnet, dass sie im Rahmen des gesamten Haarzyklus eine ausgeprägte Aktivität der alkalischen Phosphatase zeigen. Die Aktivität der alkalischen Phosphatase ist in den DSC-Zellen signifikant geringer ausgeprägt. Die DS-Zellen zeigen keine alkalischen Phosphatase-Aktivität. Im Rahmen der Erfindung bedeutet eine geringe alkalischen Phosphatase-Aktivität, dass die Aktivität der DSC-Zellen gegenüber den DP-Zellen bis zu etwa 10%, 15%, 20%, 25%, 30%, 35%, 40%, 45% oder 50% geringer ist. Ferner bedeutet eine geringe alkalischen Phosphatase-Aktivität, dass die Aktivität der DSC-Zellen mindestens etwa 10% geringer, vorzugsweise mindestens etwa 15%, 20%, 25%, 30%, 35%, 40%, 45% oder 50% geringer ist, als die ausgeprägte Aktivität der alkalischen Phosphatase in der DP.

Die vorliegende Erfindung betrifft ein Verfahren zur Isolierung von mesenchymalen Stammzellen des Haarfollikels, umfassend die folgenden Schritte:
a) Präparation vitaler Haare,
b) Spalten des in Schritt a) präparierten Haares,
c) Isolieren der tassenförmig angelagerten Haarschale zusammen mit der dermalen Haarpapille,
d) Trennen der dermalen Haarpapille von der Haarschale,
e) Kultivieren der in Schritt d) erhaltenen Haarschale,
f) Poolen der konfluenten Zellen.

Vorzugsweise stammen die Haarfollikel aus einem Säugetier, insbesondere aus einer Maus, Ratte, Kaninchen, Meerschwein, Ziege, Schwein, Rind oder Mensch. Die vorliegende Erfindung bertrifft ferner mesenchymale Stammzellen des Haarfollikels, erhältlich nach dem erfindungsgemäßen Verfahren.

Die DSC-Zellen können mit Hilfe des folgenden Verfahrens isoliert werden. Durch Mikrodissektion wird ein Haarfollikel zunächst in seine Anteile wie folgt getrennt. Dabei werden vitale Haare, z.B. intakte Anagenhaare unter dem Dissektionsmikroskop präpariert. Bei pigmentierten Haaren wird am oberen Pol der Pigmentzone ein Querschnitt durch das Haar gelegt (Fig. 3b, Pfeile) und die tassenförmig angelagerte Haarschale (DSC) zusammen mit der dermalen Haarpapille (DP) abgestreift (Fig. 3c). Dieses Gewebsstück wird umgestülpt (Fig. 3d) und die dermale Haarpapille (Fig 3e) von der Haarschale (Fig. 3f) getrennt. Dieses Verfahren kann nicht nur für die Präparation von mesenchymalen Stammzellen des Haarfollikels, sondern auch für die Präparation von mesenchymalen Stammzellen Zellen des Nagel- und des Zahnapparates angewendet werden. Das Verfahren kann bei allen eukaryontischen Organismen z.B. bei Säugetieren, insbesondere beim Menschen angewendet werden.

Die erhaltene Haartasse wird in der Zellkultur unter Standardbedingungen vermehrt. Beispielsweise kann wie folgt kultiviert werden. Eingesetzt wird als Medium AmnioMax C 100 Basalmedium (Gibco) und AmnioMax C100 Supplement. In dieses Medium werden unter sterilen Bedingungen die DSC zunächst in 24-well-Kulturschalen (Falcon, Franklin Lakes, NJ, USA) unter Standardbedingungen (37°C, 5%CO₂ 500µl Medium) kultiviert. Nach wenigen Tagen wachsen Zellen spontan aus, und werden nach Erreichen einer konfluierenden Kultur mit 200µl/Well Trypsin-EDTA abgelöst (stoppen der Trypsinierung mit 260µl Amniomax Medium/Well nach der Ablösung aller Zellen) und in 25ml Kulturflaschen (Greiner, Frickenhausen) überführt. Dazu werden die Zellen gepoolt, für 10 Minuten bei 1000 U/min zentrifugiert, der Überstand verworfen und in 5ml Amniomax aufgenommen. Das Medium wird alle 3 Tage gewechselt. Als Nachweis, dass die erhaltenen Zellen die mesenchymalen Stammzellen sind, kann eine alkalischer Phosphatase-Nachweis durchgeführt werden. Dazu können die Zellen auf sterilen Glasdeckgläschen gezüchtet, in Aceton fixiert und wie in den Beispielen beschrieben untersucht werden. Die Inkuabtionszeit des in vitro Nachweises entsprechend den beschriebenen Beispielen ist unter Standardbedingungen etwa 30 Minuten bis etwa 1,5 Stunden, vorzugsweise etwa 1 Std.

Mit dem gleichen Verfahren können sowohl die mesenchymalen Stammzellen des Nagel- als auch des Zahnapparates isoliert und vermehrt werden.

Die DSC-Zellen lassen sich mittels Zellkultur expandieren und die Zellkulturen lassen sich über mehrere Passagen züchten. Die DSC-Zellen zeigen zum einen morphologische zum anderen biochemische Eigenschaften, die sich eindeutig voneinander unterscheiden lassen. Die Fibroblasten der dermalen Bindegewebshülle (DS) zeigen morphologisch ein typisches Wachstumsmuster, das an ein fischfleischähnliches Muster erinnert. Die DSC-Zellen wachsen kompakter, bilden nicht diese fischfleischähnlichen Strukturen aus und zeigen eine Tendenz zu Bildung von sogenannten Pseudopapillen, das heißt, es bilden sich in der Zellkulturschale kleine Zellhaufen, die morphologisch an eine dermale Haarpapille erinnern. Wie bereits vorstehend erwähnt, exprimieren die Fibroblasten der follikulären Bindegewebshülle (DS) keine alkalische Phosphatase, die DSC-Zellen nur eine sehr geringe, während die DP-Zellen in vitro und in vivo eine wiederum starke Aktivität der alkalischen Phosphatase aufweisen.

Die Bindegewebszellen am unteren Pol der Bindegewebsscheide, die sog. Haartassenzellen = DSC, können sämtliche relevante, aus der Dermis entstehenden Strukturen der Haarfollikeleinheit erneuern. Durch diese Eigenschaft ermöglichen sie die Bildung von neuem Haarwachstum oder durch Repopulation einer kleinen Haarpapille die Bildung eines dickeren Haares. Die Lebensdauer der neugebildeten Haare ist dabei zeitlich nicht begrenzt sondern kann prinzipiell eine lebenslängliche sein. Eine derart lebenslange regenerative Kapazität ist dagegen nach Implantation von DP-Zellen nicht beschrieben. Alle Versuche mit DP-Zellen sind nur vorübergehender Natur, während durch die Implantation von DSC-Zellen genuine Stammzellen mit lebenslanger Proliferationsmöglichkeit in die Haut eingebracht werden.

Die betreffenden mesenchymalen Stammzellen des Haarfollikels können als Mittel für Therapie und Prophylaxe sowie zu kosmetischen Behandlunger verwendet werden. Die DSC-Stammezellen können zur Herstellung eines Mittels zur Therapie oder Prophylaxe von Alopezie oder für die Gentherapie verwendet werden.

Die mit dem erfindungsgemäßen Verfahren erhältlichen Zellen können zur Behandlung einer Alopezie insbesondere einer Alopecia areata, androgenetische Alopezie, Pseudopelade Brocq, Alopezie bei Lichen planopilaris, Lupus erythematodes, einer angeborenen Hypotrichose und Atrichien, diffusem Haarverlust im Rahmen einer Stoffwechselerkrankung, Alopezie nach Verbrennung oder Traumen oder Alopezie nach einer Chemotherapie sowie zur Gentherapie verwendet werden. Die Applikation kann z.B. durch Injektion der Zellen in Lösung (z.B. physiologischer Kochsalzlösung) oder Implantation, d.h. eingebettet in eine Matrix (z.B. Kollagen) oder liposomal verpackt erfolgen. Wenn eine einmalige Injektion oder Implanation nicht ausreicht, sind Nachbehandlungen möglich. Sind für einzelne Hautareale bestimmte Applikationsarten bevorzugt, so können die DSC-Zellen entsprechend verabreicht werden.

Die entsprechenden Eigenschaften besitzen die entsprechenden mesenchymalen Stammzellen Zellen des Nagel- und des Zahnapparates (Chuong et al, 2001, Thesleff, 2000). Eine Ursache dafür könnte darin bestehen, dass sie entwicklungsgeschichtlich gemeinsame Ursprünge haben, und da während der Embryogenese wesentliche morphologische Strukturen allen gemeinsam rekapituliert werden, findet sich die Lage der mesenchymalen Stammzellen des Haarfollikels auch bei Nägel und Zähnen wieder. Daher sind auch Zähne mit kultivierten Zellen des peribulbären Zahnapparates neu bildbar. Durch die Nutzung der morphogenen Eigenschaften kann in Analogie zum Haar, ein neuer oder dickerer Nagel oder Zahn induziert werden.

Mit der Fähigkeit der DSC-Zellen, neue Haarfollikeln zu erzeugen, oder die in der Haut schon vorhandenen dermalen Haarfollikeln zu ergänzen, ist es somit möglich, alle Formen von Haarverlust und Haarminiaturisierung zu behandeln. Dazu kommt die lange Überlebensfähigkeit der DSC-Zellen und ihre Fähigkeit, sich ohne Weiteres implantieren zu lassen und dabei voll funktionsfähig zu bleiben. Die DSC-Zellen des Haarfollikels, des Nagel oder Zahnapparates können ebenfalls Verwendung in der Gentherapie finden, bei der die Produktion von sekretorischen Stoffen erforderlich ist. Diese Zellen könnten derart genetisch modifiziert werden, dass z.B. nach Transfektion die Zellen befähigt wären, das gewünschte Produkt auszuscheiden. Über die Haut könnte das Produkt systematisch über das Blut verteilt werden. Als exemplarisches Beispiel sei hier die Transfektion der DSC-Zellen mit einem Insulingen genannt. Nach Implantation dieser nun Insulin-produzierenden Zellen wäre die Behandlung eines Diabetes mellitus möglich. Viele andere Beispiele für einen Mangel eines Sekretionsproduktes (Hormone, Proteine, Zytokine, Chemokine, Wachstumsfaktoren, Lipomediatoren) sind bekannt.

*Mikrodissektion:* Durch Mikrodissektion wurde ein Haarfollikel der Schnurhaare einer Maus zunächst in seine Anteile getrennt. Wie in der Figur 3a ersichtlich wurden zunächst intakte Anagenhaare unter dem Dissektionsmikroskop präpariert. Bei pigmentierten Haaren wurde am oberen Pol der Pigmentzone ein Querschnitt durch das Haar gelegt (Fig. 3b, Pfeile) und die tassenförmig angelagerte Haarschale (DSC) zusammen mit der dermalen Haarpapille abgestreift (Fig. 3c). Dieses Gewebsstück wurd umgestülpt (Fig. 3d) und die dermale Haarpapille (Fig 3e) von der Haartasse (Fig. 3f) getrennt. Nach der Dissektion verblieben die epithelialen Wurzelscheiden (Fig. 3h) und die Bindegewebshülle (Fig. 3i).

*Zellkultur:* Die dissezierte Haartasse wurde danach in der Zellkultur vermehrt. Eingesetzt wurde als Medium AmnioMax C 100 Basalmedium (Gibco) und AmnioMax C100 Supplement. In dieses Medium wurden unter sterilen Bedingungen die DSC zunächst in 24-well-Kulturschalen (Falcon, Franklin Lakes, NJ, USA) unter Standardbedingungen (37°C, 5%CO₂, 500µl Medium) kultiviert. Nach wenigen Tagen wuchsen Zellen spontan aus, und wurden nach Erreichen einer konfluenten Kultur mit 200µl/Well Trypsin-EDTA abgelöst (stoppen der Trypsinierung mit 260µl Amniomax Medium/Well nach der Ablösung aller Zellen) und in 25ml Kulturflaschen (Greiner, Frickenhausen) überführt. Dazu wurden die Zellen gepoolt, für 10 Minuten bei 1000 U/min zentrifugiert, der Überstand verworfen und in 5ml Amniomax aufgenommen. Das Medium wurde alle 3 Tage gewechselt. Eingesetzt wurde als Medium AmnioMax C 100 Basalmedium (Gibco) und AmnioMax C100 Supplement. In dieses Medium wurden unter sterilen Bedingungen die DSC zunächst in 24-well-Kulturschalen (Falcon, Franklin Lakes, NJ, USA) kultiviert. Nach wenigen Tagen wuchsen Zellen spontan aus, vermehrten sich und konnten mit Standardmethoden in 25ml Kulturflaschen (Greiner, Frickenhausen) subkultiviert werden.

*Detektion der alkalischen Phosphatase:* Für *in vivo* Untersuchungen wurden Gewebe tiefgefroren, in OCT-Reagens (Tissue tec, Sakura, Zoeterwounde, Niederlande) eingebettet und 6µm dicke Gefrierschnitte angefertigt. Detektiert wurde die alkalische Phosphatase mit alkalische Phosphatase "fast red TR"-Substratlösung (Fa. Pierce, Rockford, IL, USA: 10mg fast red TR wie geliefert, 10ml Substratpuffer, 1.5ml Naphthol AS-MX Phosphat-Konzentrat wie geliefert) bei pH 8.1 gemäß den Angaben des Herstellers. Entwickelt wurde für 30 Minuten in Abwesenheit von Levamisol. Für die Detektion der alkalischen Phosphatase *in vitro* wurden die Zellen auf sterilen Glasdeckgläschen gezüchtet, in Aceton fixiert und wie beschrieben das Reagens zur Messung der alkalischen Phosphatase benutzt. Die Inkuabtionszeit betrug 1 Std.

*Haarwuchsinduktion:* Nach wenigen Zellpassagen waren die Zellen noch in der Lage, ein neues Haar zu induzieren. Nach einer kleinen Verletzung (Ritz), wurden in ein Mäuseohr 3-5x10⁶ Zellen in 0,1ml PBS in die Dermis mit einer sterile 16-Gauge-Kanüle etwa 2mm neben der Wunde injiziert. Für diese Experimente wurde die Tiere narkotisiert mit 1.66ml Xylazinhydrochloride (Rompun, Bayer Vital Leverkusen, Deutschland) in 10ml Ketamin-Hydrochlorid (Hexal, Holzkirchen, Deutschland). Danach wurde im Verlauf von mehreren Wochen beobachtet, ob sich neues Haarwachstum zeigt. Nach zwei Monaten wurde Haarwuchs nach Implantation von DP und DSC beobachtet, nicht aber nach Implantation von DS-Zellen. Der Haarwuchs setzte sich über den Zeitraum von 6 Monaten fort, was darauf hinweist, dass diese klinischen Beobachtungen kein vorübergehendes Phänomen sind. Weiterhin konnte beobachtet werden, dass bereits vorhandene Haare nach Implantation dicker wurden (Fig. 6). *Confocale Lasermikoskopie:* Neben der biologischen Eigenschaft der Haarwuchsinduktion durch DSC-Zellen wurde die Wanderung der unterschiedlichen Zellen nach Implantation beobachtet Hierfür wurden aus den drei beschriebenen morphologischen Haarzonen (DP, DS, DSC) von Mäusen (STOCK TgN(GFPX)4Nagy) Gewebe präpariert. Diese Mäuse wurden gewählt, da alle kernhaltigen Zellen dieser Mäuse grün fluoreszierende Proteine tragen. Aus diesen Geweben wurden Zellen kultiviert und über einen Zeitraum von 6 Wochen passagiert. Die drei unterschiedlichen Zellenarten wurden in die Ohren von immuninkompetenten CbySmm.CB17-*Pi-kdc^{scid}*/J Mäusen injiziert. Zusätzlich wurden Zellen von nicht-fluoreszierenden, GFP STOCK TgN(GFPX) 4Nagy, C3H/HeJ Mäuse und PVG/OlaHsd Ratten in gleicher Weise injiziert. Beobachtet wurde, ob sich Haarneuwachstum oder eine Verdickung bereits bestehender Haare zeigt. Nach 2-6 Monaten wurden die Tiere getötet und die Ohren eingebettet. Hierfür wurden die Gewebe in 4%-igen Paraformaldehyd (Sigma, Deisenhofen) in PBS^{-/-} für 2 Std fixiert, danach mit PBS^{-/-} mehrfach gespült. Das Gewebe wurde in "Tissue Tek" bei Raumtemperatur einbettet und 24 Stunden dunkel bei + 4° C gelagert. Anschliessend wurden die Gewebe in einer mit Zellstoff ausgepolsterten Styroporbox langsam auf - 70° C heruntergekühl (sog. "slow freezing technique"). Der Gewebeblock wurden zunächst 30 Minuten auf- 20° C erwärmt. Im Kryostaten wurden dann Schnitte zwischen 20 und 40 µm hergestellt, und die auf mit 1% Poly-L-Lysin (Sigma, Deisenhofen, Deutschland) vorbehandelten Objektträgern aufgezogen werden. Das Trocknen erfolgte bei Raumtemperatur. Danach wurden die Schnitte 1x mit PBS^{-/-} gespühlt, bevor die Schnitte mit PBS^{-/-}- oder wasserhaltigem Eindeckmedium, z. B. Glycerol, eingedeckt wurden. Danach wurden die Gewebe mit einem Lasermikroskop der FA. Zeiss (Göttingen, Deutschland), Typ LSM 410, bei den Wellenlängen von 488 nm Exzitation, 500 - 520 nm Emission, Z-Achse in 2 µm-Intervallen mit dem Argon-Krypton-Laser aufgenommen.

In der Folge wurde Serienschnitte (20-40µm) angefertigt und im konfokalen Lasermikroskop auf das Vorhandensein von GFP (= green fluoreszent protein)-exprimierenden Zellen untersucht. Mit diesen Versuchen konnten Voruntersuchungen bestätigt werden, dass durch Implantation von DP-Zellen Haarneuwachstum möglich ist und dass nur die implantierten Zellen das neue Haar bilden, während implantierte DS-Zellen nicht zur Haarbildung führten, wohl aber im konfokalen Mikroskop als diffuse, GFP-exprimierende Zellpopulation in der Dermis sichtbar war. Implantierte DP-Zellen führten nur zur Bildung einer neuen dermalen Haarpapille, nicht aber zur Bildung einer follikulären Bindegewebshülle. Die DSC-Zellen hingegen bildeten sowohl eine neue dermale Haarpapille als auch einen Teil der follikulären Bindegewebshülle. Dadurch dass die Haartassenzellen alle dermalen Haarstrukturen erneuern können, während Zellen der dermalen Haarpapille dies nicht mehr können, ist abzuleiten, dass die DSC etwas undifferenzierter und pluripotenter als Zellen der dermalen Haarpapille sind. Damit sind die DSC-Zellen die adulten mesenchymalen Stammzellen des Haarfollikels. In der Fig. 4 ist die induktive Eigenschaft der DSC-Zellen verdeutlicht. Zusammenfasend konnte als Ergebnis beobachtet werden, dass die DSC die mutmaßlichen Stammzellen sind, aus denen sich die dermale Papille und die follikuläre Bindegewebsscheide bilden.

Es konnte gezeigt, dass nach Implantation von DSC sich ein neues Haar bildet und dass implantierte DSC-Zellen sowohl eine neue dermale Papille als auch eine neue Bindegewebshülle bilden. Im konfokalen Mikroskop war dies sichtbar durch Grünfluoreszenz sowohl in der Zone der Haarschale, als auch der dermalen Papille und der Bindegewebsscheide. Aus der Analyse, die sechs Monate nach Injektion von Zellen gemacht wurde, ging hervor, dass GFP-exprimierende Zellen in den relevanten Haarfollikelstrukturen immer noch anwesend waren. Die injizierten Zellen haben einen für Stammzellen typischen sehr langsamen Zellzyklus und weisen eine regenerative Kapazität auf. Es gab keine Rekrutierung von nicht-GFP-exprimierenden dermalen Zellen von Seiten des Wirtes. Weiterhin konnten einzelne grün fluoreszierende Zellen in vorbestehenden Haare beobachten werden, als Indiz dafür, dass implantierte DSC-Zellen bestehende dermale Haarpapille besiedeln und dadurch zu einem dickeren Haar führen.

### Literatur

Chuong CM, Hou L, Chen PJ, Wu P, Patel N, Chen Y (2001) Dinosaur's feather and chicken tooth? Tissue engineering of the integument. Eur J Dermatol 11:286-292.
Hutchinson PE, Thompson JR (1997) The cross-sectional size and shape of human terminal scalp hair. Br J Dermatol 136:159-165
Hutchinson PE, Thompson JR (1999) The size and form of the medulla of human scalp hair is regulated by the hair cycle and cross-sectional size of the hair shaft. Br J Dermatol 140:438-445
Jahoda CA, Horne KA, Oliver RF. Induction of hair growth by implantation of cultured dermal papilla cells. Nature. 1984 Oct 11-17;311(5986):560-2.
Kligman A (1959) The human hair cycle. J Invest Dermatol 31:307-316
Messenger AG. The culture of dermal papilla cells from human hair follicles. Br J Dermatol. 1984 Jun;110(6):685-9.
Oliver RF. The induction of hair follicle formation in the adult hooded rat by vibrissa dermal papillae. J Embryol Exp Morphol. 1970 Feb;23(1):219-36.Paus R, Cotsarelis G (1999) The biology ofhair follicles. N Engl J Med 341:491-497
Reynolds AJ, Lawrence C, Cserhalmi-Friedman PB, Christiano AM, Jahoda CA. Trans-gender induction of hair follicles. Nature. 1999 Nov 4;402(6757):33-4.
Sperling LC (1991) Hair anatomy for the clinician. J Am Acad Dermatol 25:1-17
Thesleff I (2000) Genetic basis of tooth development and dental defects. Acta Odontol Scand 58:191-194.

## Patentansprüche

1. Verfahren zur Isolierung von mesenchymalen Stammzellen des Haarfollikels, umfassend die folgenden Schritte:
a) Spalten eines zuvor präparierten Haares,
b) Isolieren der tassenförmig angelagerten Haarschale zusammen mit der dermalen Haarpapille,
c) Trennen der dermalen Haarpapille von der Haarschale,
d) Kultivieren der in Schritt c) erhaltenen Haarschale zu mesenchymalen Stammzellen, wobei die mesenchymalen Stammzellen eine alkalische Phosphatase-Aktivität aufweisen,
e) Poolen der konfluenten Zellen.

2. Verfahren nach Anspruch 1, wobei der Haarfollikel aus einem Säugetier stammt.

3. Verfahren nach Anspruch 2, wobei das Säugetier eine Maus, Ratte, Kaninchen, Meerschwein, Ziege, Schwein, Rind oder Mensch ist.

## Claims

1. A method for isolating hair follicle mesenchymal stem cells, the method comprising the following steps:
a) cleavage of a hair previously prepared,
b) isolating the cup shape-like attached hair cup together with the dermal hair papilla,
c) separating the dermal hair papilla from the hair cup,
d) cultivating the hair cup obtained in step c) to mesenchymal stem cells, wherein the mesenchymal stem cells exhibit a alkaline phosphatase activity,
e) pooling of the confluent cells.

2. The method according to claim 1, wherein the hair follicle is derived from a mammal.

3. The method according to claim 2, wherein in the mammal is a mouse, a rat, a rabbit, a guinea pig, a goat, a pig, a bovine or a human.

## Revendications

1. Procédé d'isolement de cellules souches mésenchymateuses du follicule pileux, comprenant les étapes suivantes :
a) fission d'un poil préparé au préalable,
b) isolement du bulbe pileux implanté sous la forme d'une tasse avec la papille pileuse dermique,
c) séparation de la papille pileuse dermique et du bulbe pileux,
d) culture du bulbe obtenu à l'étape c) pour donner des cellules souches mésenchymateuses, les cellules souches mésenchymateuses présentant une activité de phosphatase alcaline,
e) regroupement des cellules confluentes.

2. Procédé selon la revendication 1, dans lequel le follicule pileux provient d'un mammifère.

3. Procédé selon la revendication 2, dans lequel le mammifère est une souris, un rat, un lapin, un cochon d'Inde, une chèvre, un cochon, un boeuf ou un homme.
